# EUROPEAN PATENT SPECIFICATION

(11) **EP 2 792 738 B1**
(45) Date of publication and mention of the grant of the patent: **01.03.2017**
(21) Application number: 11877381.1
(22) Date of filing: 14.12.2011
(51) Int. Cl.: C12M 1/00, C12N 5/07, C12N 15/09

(54) **CELL CULTURE CONTAINER, AND AUTOMATED CELL SUBCULTURE DEVICE AND CELL SUBCULTURE METHOD USING SAME**
ZELLKULTURBEHÄLTER, UND AUTOMATISIERTE ZELLSUBKULTURSVORRICHTUNG SOWIE ZELLSUBKULTURVERFAHREN DAMIT
RÉCIPIENT DE CULTURE CELLULAIRE, ET DISPOSITIF DE SOUS-CULTURE CELLULAIRE AUTOMATISÉ ET PROCÉDÉ DE SOUS-CULTURE CELLULAIRE L'UTILISANT

(43) Date of publication of application: 22.10.2014
(73) Proprietor: Hitachi, Ltd., Tokyo 100-8280 (JP)
(72) Inventor: ZHOU Guangbin, Tokyo 100-8280 (JP); MATSUOKA Shizu, Tokyo 100-8280 (JP)
(74) Representative: MERH-IP Matias Erny Reichl Hoffmann Patentanwälte PartG mbB
(86) International application number: PCT/JP2011/078966
(87) International publication number: WO 2013/088537

(56) References cited:
- WO-A1-2008/136371
- WO-A1-2009/123173
- WO-A1-2009/123173
- WO-A1-2011/142670
- JP-A- 2000 125 848
- JP-A- 2004 089 136
- JP-A- 2004 089 136
- JP-A- 2004 097 047

## Description

### Technical Field

The present invention relates to a cell culture container, an automated cell subculture device and a cell subculture method which perform subculture of various cells.

### Background Art

Heretofore, subculture of various cells (floating cells, adherent cells) has been performed. In particular, anchorage-dependent cells are adhered to the bottom surface (culture surface) of the culture container and proliferated, and therefore it is necessary to pay attention to always maintaining the cell density within a constant range. If the cell density is too low and the distance between neighboring cells is too large, anchorage-dependent cells may die because of lowered proliferation rate. On the other hand, if the cell density is too high and the distance between neighboring cells is too small, the anchorage-dependent cells may stop proliferating and die in that state. Therefore, when culturing the anchorage-dependent cells, they need to be sequentially subcultured into a culture container.

In a subculture at an early stage of culture, incubation is performed in a culture container with a small culture zone, so that the density of anchorage-dependent cells is not too low. To this end, when the anchorage-dependent cells proliferate to a certain degree, they are subcultured into a culture container with a greater culture zone, or a plurality of culture containers having the same culture zone. This operation is repeated until the anchorage-dependent cells are proliferated to the target number of cells. Heretofore, in the cell culture operation involving such a subculture, the cells in the primary culture container need to be dispensed into a plurality of subculture containers by a skilled operator using a pipette. Heretofore, this operation has been disadvantageously troublesome.

Patent Literature 1 proposes a cell culture device in which a first culture unit and a second culture unit are connected by piping, and a culture medium exchange operation and a subculture operation are performed in a timely manner and automatically by a cell culture program. Moreover, Patent Literature 2 proposes a culture container having culture zones partitioned by a fixed partition piece for primary culture in a subculture container and an automatic subculture device.

In JP 2004 089136 A a cultural vessel for culturing a cell growing along a bottom surface and which can enlarge the area of the bottom surface for growing the cell is described. It is thereby an object to keep cultural environment of a mesenchymal stem cell by a simple vessel and efficiently proliferate the cell.

In WO 2009/123173 A1 a culture container is described. The culture container for culturing cells in which cells and a medium are enclosed comprises at least one channel connecting the front section of a storage part to the rear section thereof. The storage part is divided into the front and rear sections with a partition member. One of the front and rear sections is employed as a culture part for conducting the culture, while the other is employed as a medium-supply part for containing the medium for replacement that is to be supplied to the culture part.

### Citation List

### Patent Literature

Patent Literature 1: Japanese Unexamined Patent Publication No. 2001-275659
Patent Literature 2: Japanese Unexamined Patent Publication No. 2006-6219

### Summary of Invention

### Problems that the Invention is to Solve

However, in the cell culture devices described in Patent Literature 1 and Patent Literature 2, when cells are subcultured, a cell suspension is transferred to a subculture zone using a transfer pump, which has posed the problems of the loss of cells and the stress exerted on the cells associated with transferring, and complication of the structure of the device. Moreover, in the fixed partition structure of Patent Literature 2, uniformly inoculating cells during the subculture is difficult.

An object of the present invention is to provide a cell culture container, an automatic subculture device and a cell subculture method which is capable of performing subculturing of cells without performing a dispensing operation using a pipette, and of preventing microbial contamination in the culture container during cell subculture.

### Means for Solving the Problems

Typical examples of the inventions disclosed in the present application are as follows:
The cell culture container of the present invention includes a first partition member having a wall surface formed to be lower than a wall surface of the culture container which forms a second culture zone in a sealable culture container which forms a first culture zone, and has means for raising and lowering the first partition member in a state that the culture container is sealed.

Moreover, the cell culture container of the present invention further includes a second partition member having a wall surface formed to be lower than a wall surface of the culture container which forms a third culture zone in the second culture zone, and has means for raising and lowering the second partition member in a state that the culture container is sealed.

Moreover, the cell culture container of the present invention further includes a third partition member having a wall surface formed to be lower than a wall surface of the culture container which forms a third culture zone which does not overlap the second culture zone, and has means for raising and lowering the third partition member in a state that the culture container is sealed.

### Advantageous Effects of Invention

According to the present invention, the subculture efficiency of the cell culture container can be improved, and microbial contamination in the culture container can be prevented during cell subculture.

### Brief Description of Drawings

Fig. 1 is a drawing which shows a constitutional example of a cell culture container of Example 1 of the present invention.
Fig. 2 is a drawing which shows a subculture by the cell culture container of Example 1 of the present invention.
Fig. 3 is a drawing which shows the principle of magnets.
Fig. 4 is a drawing which shows a variant of the cell culture container of Example 1 of the present invention.
Fig. 5 is a drawing which shows a constitutional example of the cell culture container of Example 2 of the present invention.
Fig. 6 is a drawing which shows a constitutional example of the cell culture container of Example 3 of the present invention.
Fig. 7 is a drawing which shows a subculture by the cell culture container of Example 3 of the present invention.
Fig. 8 is a drawing which shows a constitutional example of the cell culture container of Example 4 of the present invention.
Fig. 9 is a drawing which shows a cell culture by the cell co-culture container of Example 4 of the present invention.
Fig. 10 is a drawing which shows an automatic cell subculture system of Example 5 of the present invention.
Fig. 11 is a drawing which shows the control flowchart of the automatic cell subculture system of Example 5 of the present invention.

### Description of Embodiments

The embodiments of the present invention will be described with reference to drawings. In each of the drawings, the identical components are given the identical numbers, and repeated explanation is omitted.

### Example 1

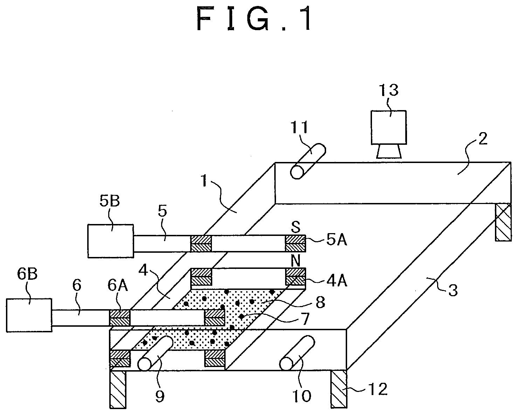

One constitutional example of the cell culture container of Example 1 of the present invention will be described with reference to Fig. 1.

In Fig. 1, 1 is a cell culture container, 2 is a ceiling substrate of the culture container 1, and 3 is a bottom surface substrate of the culture container 1. The area of the bottom surface substrate 3 partitioned by the frame of the culture container 1 is a first culture zone (subculture zone). 4 is a movable partition, and 4A is a magnet fitted into the movable partition 4. The movable partition 4 is lower than the wall surface of the culture container 1, and the region the bottom surface substrate 3 of the partitioned culture container 1 is a second culture zone (primary culture zone). 5 and 6 are supporting mechanisms which are located outside the ceiling substrate of the culture container 1, 5A and 6A are magnets which are fitted into the supporting mechanisms 5, 6, respectively, and 5B and 6B are rotation mechanisms which rotate the supporting mechanisms 5, 6, respectively. 7 is a first target cell, 8 is a culture medium which provides nutrient compositions of cells. 9 is the inlet and outlet of the culture medium 8, 10 is the inlet of mixed gas (air including 5% of CO₂, and at humidity of 90% or higher), and 11 is the outlet for mixed gas. 12 is a drive mechanism for shaking the culture container 1 for uniform inoculation and cell peeling as well as for inclining the culture container 1 for discharging culture medium and the like. 13 is an observation mechanism for observing cells.

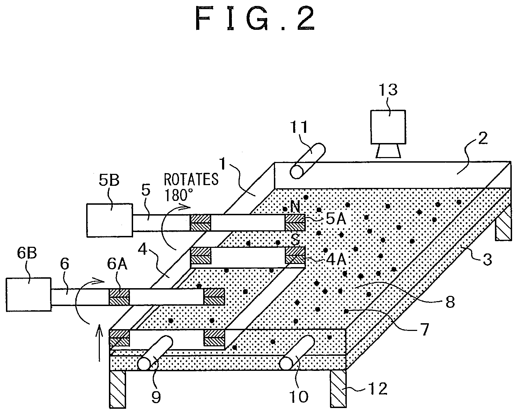

The flow of the subculture of this example will be described below with reference to Figs. 1 and 2. First, in the primary culture of target cells, as shown in Fig. 1, after the culture medium 8 is provided from a culture medium inlet outlet 9 to the primary culture zone in the movable partition 4, the suspension of the target cell is poured into the container. At this time, the magnets 5A, 6A in the supporting mechanisms 5, 6 are in such a state that they face the magnet 4A in the movable partition 4 with the same poles facing each other (N-pole against N-pole), and the movable partition 4 is closely fitted to the bottom surface substrate 3. In this state, the entire culture container 1 can be shaken by the drive mechanism 12 to uniformly inoculate the cells. In addition, a mixed gas (air containing 5% of CO₂, and at humidity of 90% or higher) required for cell culture is supplied from a mixed gas inlet 10 into the culture container 1. Since the movable partition 4 is lower than the wall surface of the culture container 1, the mixed gas can be supplied to the primary culture zone. The culture process of cells are observed and measured by the observation mechanism 13.

Around the time when the cell proliferation limit is reached on the primary culture surface, the culture medium of the primary culture zone is discharged, and the cells are washed with PBS (physiological saline). Trypsin is then poured into the primary culture zone to remove the cells off from the culture surface, and the culture medium is then poured to cause the cells to float in the culture medium. Next, as shown in Fig. 2, the supporting mechanisms 5, 6 are rotated 180° by the rotation mechanisms 5B, 6B, so that the magnets 5A, 6A in the supporting mechanisms 5, 6 face the magnet 4A in the movable partition 4 with their different poles facing each other (S-pole against N-pole). As a result, by the attractive force of the magnets, the movable partition 4 is detached from the bottom surface substrate 3 by the supporting mechanisms 5, 6 located on the outside of the culture container, and is raised to the ceiling substrate 2 of the culture container. The partition 4 detaches from the bottom surface substrate 3, whereby a gap is produced between the partition 4 and the bottom surface substrate 3, and the cell suspension which is in the primary culture zone is diffused in the subculture zone of the cell culture container 1 having a culture surface greater than the primary culture zone. In addition, a culture medium which is suitable for the culture zone is poured into the subculture zone from the culture medium inlet outlet 9, and the cells are uniformly dispersed into the subculture zone by shaking with the drive mechanism 12. After the cells are adhered to the culture surface, the culture medium is exchanged to remove trypsin and subculturing is then performed. After the subculturing, a cell suspension containing more cells can be collected by an operation similar to the process described above.

The above-mentioned non-contact subculture operation in a closed system culture container is realized by using the magnet mechanism, and microbial contamination in the culture container during subculturing can be prevented. In addition, during the subculturing operation, since a transfer pump is not required, no loss of cells or stress on cells associated with transferring by the pump occurs.

For the components of the movable partition 4 stated above, it is desirable to use materials suitable for cell culture such as polycarbon and polystyrene.

The ceiling substrate 2 and the bottom surface substrate 3 of the culture container 1 stated above can be formed from base materials of solid substrates such as glass, silicon halides, quartz, or plastics and polymers. More desirably, these substrates have such optical transparency that can be observed by an optical microscope and other means, and further the bottom surface substrate 3 desirably has a material on which cleaning can be performed before depositing cells onto the surface and surface reforming of the substrate by preprocessing.

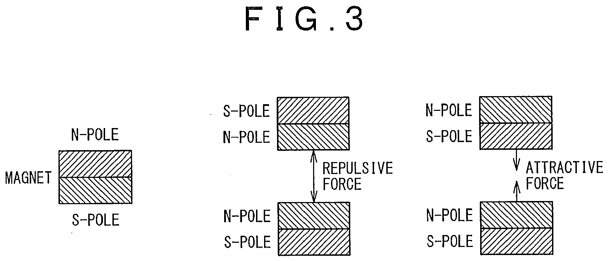

In this example, the magnets are used as means for raising and lowering the partition member. The principle of transfer using the magnets will be described with reference to Fig. 3. A magnet is a substance which has two magnetic poles (N-pole, S-pole) and serves as a source of production of a bipolar magnetic field (on the left hand of Fig. 3). The magnetic poles of the magnet do not exist solely, but both poles always constitute a magnet together. When two magnets are approached to each other, a force which attracts each other (attractive force) acts between different poles (on the right hand of Fig. 3), while a force which repels each other (repulsive force) acts between the same poles (at the center of Fig. 3). In addition to permanent magnets (ferrite magnets, neodymium magnets, etc.), electromagnets which temporarily produce a magnetic force by energized coils are also available.

It should be noted that in the above example, the configurations of the outer frame and partition of the culture container being square have been described, but it goes without saying that they may be circular, polygonal or in other shapes.

Moreover, in this example, it has been described that the supporting mechanisms 5, 6 which raise and lower the partition 4 in the culture container 1 are provided in an upper part of the ceiling substrate, but the supporting mechanisms 5, 6 may be provided on the outside of the side face of the culture container 1. In this case, it can be realized by providing a vertical moving mechanism which vertically moves the magnets 5A, 6A in place of the rotation mechanism.

In addition, a hole may be provided in the culture container 1, the supporting member may be connected with the partition 4 through this hole, and the partition 4 may be moved by moving this supporting member. In this case, the sealing property between the hole and supporting member needs to be ensured.

In addition, in this example, the subculture by means of the culture container by using the subculture of adherent cells has been explained, but the culture container of this example can also be applied to the subculture of floating cells.

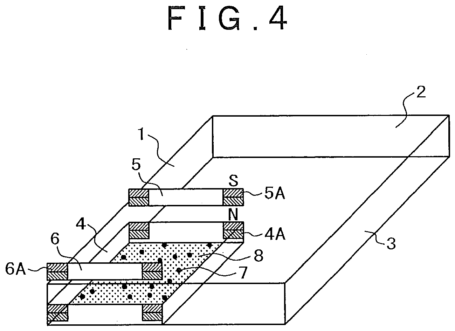

Moreover, in this example, it has been stated that the operation from the primary culture to subculture is automatically performed, but as shown in Fig. 4, the culture container of this example can also be applied to subculture by manual operation.

The cell subculture method of Example 1 is a cell subculture method which uses a cell culture container including a partition member having a wall surface formed to be lower than a wall surface of the culture container which forms the second culture zone in a sealable culture container which forms a first culture zone, and having means for raising and lowering the partition member in a state that the culture container is sealed, the method including the steps of culturing cells in the second culture zone in a state that the partition member is lowered, raising the partition member, and dispersing the cells cultured in the second culture zone into the first culture zone, and culturing cells in the first culture zone and the second culture zone.

According to this example, the subculture efficiency of the cell culture container can be improved, and microbial contamination in the culture container can be prevented during cell subculture.

### Example 2

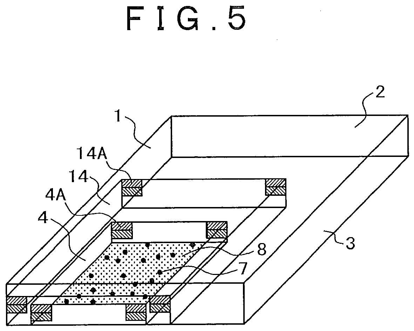

Fig. 5 shows a constitutional example of the cell culture container of Example 2 of the present invention. Example 2 is a cell culture container with a two-passage structure.

In Fig. 5, the parts other than a movable partition 14 and a magnet 14A fitted into the movable partition 14 are similar to those in Example 1.

The culture container of this example includes a movable partition 4 having a wall surface formed to be lower than a wall surface of a culture container 1 which forms a third culture zone (primary culture zone) in the sealable culture container 1 which forms a first culture zone (second-passage subculture zone), has means for raising and lowering the partition in a sealing state, and includes the movable partition 14 having the wall surface formed to be lower than the wall surface of the culture container 1 which forms a second culture zone (first passage subculture zone) in the first culture zone, and has means for raising and lowering the partition in a state that the culture container 1 is sealed.

The cell subculture method of Example 2 is a cell subculture method which uses a cell culture container which includes, in a sealable culture container which forms a first culture zone, a first partition member having a wall surface formed to be lower than a wall surface of the culture container which forms a second culture zone, has means for raising and lowering the first partition member in a state that the culture container is sealed, and further includes a second partition member having a wall surface formed to be lower than a wall surface of the culture container which forms a third culture zone in the second culture zone, and having means for raising and lowering the second partition member in a state that the culture container is sealed, the method including a step of culturing cells in the third culture zone in a state that the second partition member is lowered, a step of raising the second partition member in a state that the first partition member is lowered and a dispersing the cells cultured in the third culture zone into the second culture zone, a step of culturing cells in the third culture zone and the second culture zone in a state that the first partition member is lowered, a step of raising the first partition member and dispersing the cells cultured in the third culture zone and the second culture zone into the first culture zone, and a step of culturing cells in the first culture zone and the second culture zone and the third culture zone.

The culture container of this example is capable of performing a primary culture and a two-passage subculture in a single closed section, and of preventing microbial contamination in the culture container during subculturing. In addition, during the subculturing operation, since a transfer pump is not required, no loss of cells or stress on cells associated with transferring by the pump occurs.

### Example 3

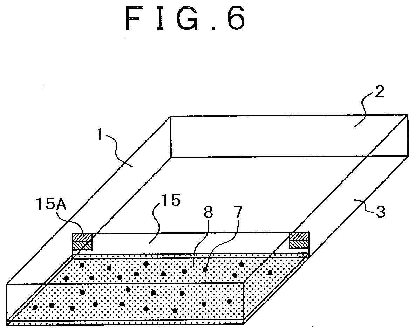

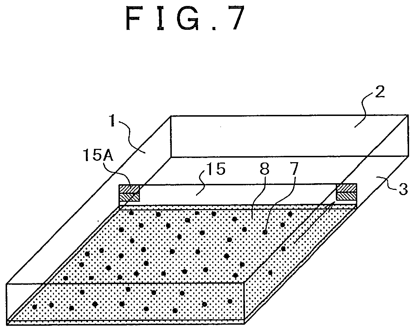

With reference to Figs. 6 and 7, the cell culture container of Example 3 of the present invention will be described. Example 3 includes a partition with a straight-line partition structure.

Herein, the parts other than a movable partition 15 of Figs. 6 and 7 and a magnet 15A fitted into the movable partition 15 are similar to those in Example 1.

The culture container of this example, as shown in Fig. 6, after the primary culture is performed in the primary culture zone with a small area in the frame of the culture container 1, depending on the cell subculture, as shown in Fig. 7, the movable partition 15 is moved to an appropriate position to ensure a cell subculture zone. The moving of the movable partition 15 may be performed by the magnets provided on the outside of the culture container as shown in Example 1, or a hole may be provided on the side face of the culture container, a supporting member may be connected with the movable partition 15 through this hole, and the movable partition 15 may be moved by moving this supporting member.

According to this structure, a culture zone for the primary culture and subcultures (several passages allowed) can be freely provided depending on cell types and inoculation concentrations, and the non-contact operation in the closed system culture container can be realized, so that microbial contamination in the culture container during subculturing is prevented. In addition, during the subculturing operation, since a transfer pump is not required, no loss of cells or stress on cells associated with transferring by the pump occurs.

The cell subculture method of Example 3 is a cell subculture method which includes a partition member which forms a culture zone into the sealable culture container, and uses a cell culture container having means for moving the partition member in a state that the culture container is sealed and changing the area of the culture zone, the method including a step of locating the partition member in an initial position to culture cells in the culture zone, a step of moving the partition member to expand the area of the culture zone, and a step of culturing cells in the culture zone with the area thereof expanded.

### Example 4

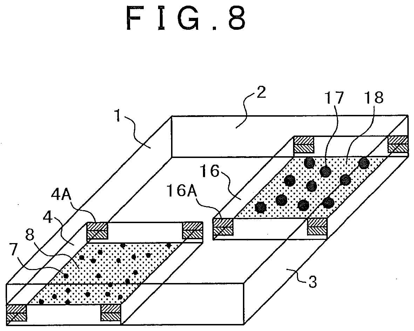

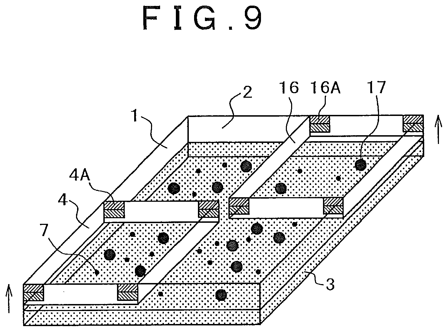

With reference to Figs. 8 and 9, the cell culture container of Example 4 of the present invention will be described. Example 4 relates to a cell culture container of with a co-culture structure.

Herein, the parts other than the movable partition 16, a magnet 16A fitted into the movable partition 16, a second target cell 17, and a culture medium 18 for second target cell in Figs. 8 and 9 are similar to those in Example 1.

The culture container of this example, as shown in Fig. 8, using the movable partition 4 and the movable partition 16, the first target cell and second target cell are cultured in the respective primary culture zones in the frame of the culture container 1, and when subculturing, as shown in Fig. 9, the movable partition 4 and the movable partition 16 are opened to perform co-culturing, so that cell incubation in which both cells can promote the proliferation of each other can be realized.

The cell subculture method of Example 4 is a cell subculture method which uses a cell culture container which includes, in a sealable culture container which forms a first culture zone, a first partition member having a wall surface formed to be lower than a wall surface of the culture container which forms a second culture zone, has means for raising and lowering the first partition member in a state that the culture container is sealed, and further includes a third partition member having a wall surface formed to be lower than a wall surface of the culture container which forms a third culture zone which does not overlap the second culture zone, and has means for raising and lowering the third partition member in a state that the culture container is sealed, the method including a step of culturing cells in the second culture zone and the third culture zone in a state that the first partition member and the third are lowered, a step of raising the first partition member and the third partition member and dispersing the cells cultured in the second culture zone and the third culture zone into the first culture zone, and a step of culturing cells in the first culture zone and the second culture zone and the third culture zone.

### Example 5

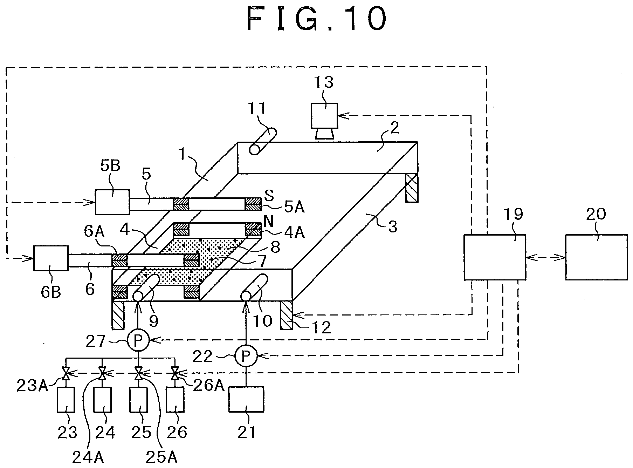

An automatic cell subculture system of Example 5 of the present invention will be described with reference to Fig. 10. The same parts as in the above examples will be referred to by the same numerals below, and their explanation will be omitted, while only different parts will be described.

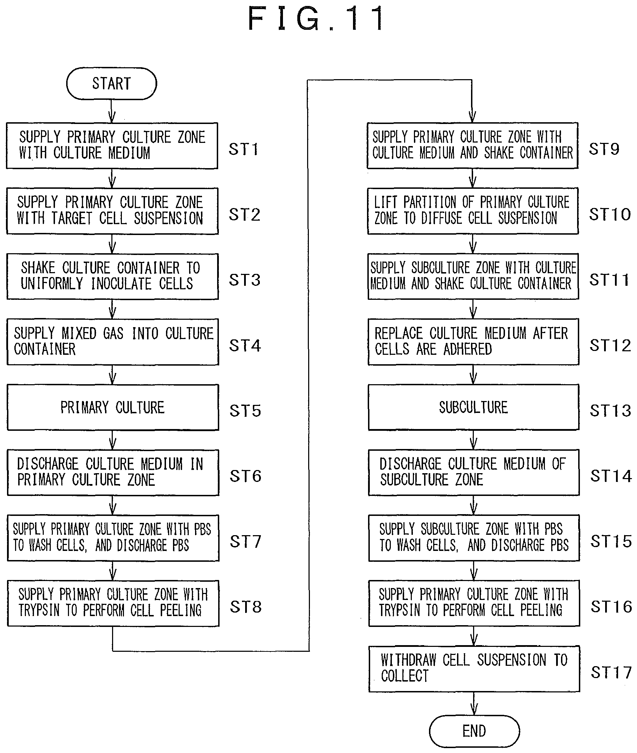

Herein, 19 is a control processor, and 20 is a monitor. 21 is a mixed gas producing device, 22 is a gas pump, 23 is a culture cell suspension tank, 24 is a culture medium tank, 25 is a trypsin tank, 26 is a PBS tank, 23A, 24A, 25A, 26A are electromagnetic valves connected to the corresponding tanks, respectively, and 27 is a liquid pump. It should be noted that the broken lines in Fig. 10 are electric signal lines connected to the control processor 19 and the respective electric control parts. The control flowchart of the same is shown in Fig. 11.

First, in the primary culture of target cells, as shown in Fig. 1, after the culture medium 8 is provided from a culture medium inlet outlet 9 to the primary culture zone in the movable partition 4 (ST1), the suspension of the target cells is poured from the culture cell suspension tank (ST2). At this time, the magnets 5A, 6A in the supporting mechanisms 5, 6 are in such a state that they face the magnet 4A in the movable partition 4 with the same poles facing each other (N-pole against N-pole). The entire culture container is shaken by the drive mechanism 12 to uniformly inoculate cells (ST3). In addition, a mixed gas (air containing 5% of CO₂, and at humidity of 90% or higher) required for cell culture is supplied from a mixed gas inlet 10 into the culture container (ST4), to perform primary culture (ST5). Since the movable partition 4 is lower than the wall surface of the culture container 1, the mixed gas can be supplied to the primary culture zone. The culture process of the cells is observed and measured by the observation mechanism 13. Around the time when the cell proliferation limit is reach on the primary culture surface, the culture medium of the primary culture zone is discharged (ST6), and PBS (physiological saline) is poured from the PBS tank 26 to wash the cells (ST7). Trypsin is then poured into the primary culture zone from the trypsin tank 25 to remove the cells off from the culture surface (ST8), and the culture medium is then poured to cause the cells to float in the culture medium (ST9).

Next, as shown in Fig. 2, the supporting mechanisms 5, 6 are rotated 180° by the rotation mechanisms 5B, 6B, so that the magnets 5A, 6A in the supporting mechanisms 5, 6 face the magnet 4A in the movable partition 4 with their different poles facing each other (S-pole against N-pole). As a result, by the attractive force of the magnets, the movable partition 4 is detached from the bottom surface substrate 3 by the supporting mechanisms 5, 6 located on the outside of the culture container and is raised to the ceiling substrate 2 of the culture container. The partition 4 is detached from the bottom surface substrate 3, whereby a gap is produced between the partition 4 and the bottom surface substrate 3, and the cell suspension which is in the primary culture zone is diffused in the subculture zone of the cell culture container 1 having a culture surface greater than the primary culture zone (ST10). In addition, a culture medium which is suitable for the culture zone is poured into the subculture zone from the culture medium inlet outlet 9, and the cells are uniformly dispersed into the subculture zone by shaking with the drive mechanism 12 (ST11). After the cells are adhered to the culture surface, the culture medium is exchanged (ST12) to perform subculturing (ST13) so as to remove trypsin. After the subculturing, a cell suspension containing more cells can be collected by an operation similar to the process described above (ST17).

An example of cell culture using Example 1 will be described. A culture container having a similar structure to that in Example 1 with the area of a primary culture zone of 100 cm² and a subculture zone of 1000 cm² was used. Moreover, the cells used were 3T3 cells (fibroblast culture cell strain derived from mouse skin), and the culture medium used was DMEM with calf serum and an antibiotic added to it. It should be noted that the inoculation density of the 3T3 cells is 2×10³ cells/cm².

Primary culture was performed for three days by the procedure of Example 1, and about 2×10⁶ cells were confirmed from the culture surface. In addition, a subculture was performed for three days and about 2×10⁷ cells could be collected from the culture surface.

The present invention is not limited to the above-described Examples unless the features of the present invention are damaged, and other forms which are conceivable within the scope the technical ideas of the present invention are also included in the scope of the present invention.

### Reference Signs List

- 1: Culture container
- 2: Ceiling substrate of Culture container
- 3: Bottom surface substrate of culture container
- 4: Movable partition
- 4A: Magnet
- 5: Supporting mechanism
- 5A: Magnet
- 5B: Rotation mechanism
- 6: Supporting mechanism
- 6A: Magnet
- 6B: Rotation mechanism
- 7: First target cell
- 8: Culture medium
- 9: Culture medium inlet and outlet
- 10: Mixed gas inlet
- 11: Mixed gas outlet
- 12: Drive mechanism
- 13: Observation mechanism
- 14: Movable partition
- 14A: Magnet
- 15: Movable partition
- 15A: Magnet
- 16: Movable partition
- 16A: Magnet
- 17: Second target cell
- 18: Culture medium for second target cell
- 19: Control processor
- 20: Monitor
- 21: Mixed gas producing device
- 22: Gas pump
- 23: Culture cell suspension tank
- 23A: Electromagnetic valve
- 24: Culture medium tank
- 24A: Electromagnetic valve
- 25: Trypsin tank
- 25A: Electromagnetic valve
- 26: PBS tank
- 26A: Electromagnetic valve
- 27: Liquid pump

## Claims

1. A cell culture container comprising:
in a sealable culture container (1) which forms a first culture zone,
a first partition member having a wall surface formed to be lower than a wall surface of the culture container (1) which forms a second culture zone; and
means for raising and lowering the first partition member in a state that the culture container (1) is sealed.

2. The cell culture container according to claim 1, further comprising:
a second partition member having a wall surface formed to be lower than a wall surface of the culture container (1) which forms a third culture zone in the second culture zone; and
means for raising and lowering the second partition member in a state that the culture container (1) is sealed.

3. The cell culture container according to claim 1, further comprising:
a third partition member having a wall surface formed to be lower than a wall surface of the culture container (1) which forms the third culture zone which does not overlap the second culture zone; and
means for raising and lowering the third partition member in a state that the culture container (1) is sealed.

4. The cell culture container according to any one of claims 1 to 3,
wherein magnets (4A, 6A, 14A, 15A, 16A) are used as means for raising and lowering the partition member.

5. The cell culture container according to any one of claims 1 to 3,
wherein a supporting member connected to the partition member is used as means for raising and lowering the partition member.

6. An automatic subculture device, comprising:
the cell culture container (1) according to any one of claims 1 to 5;
a culture medium supply mechanism which supplies a culture medium to the culture container (1);
a mixed gas supply mechanism which supplies a mixed gas into the culture container (1); and
a cell observation mechanism for observing a culture surface of the culture container (1).

7. A cell subculture method which uses the cell culture container (1) according to claim 1, the method comprising:
a step of culturing cells in the second culture zone in a state that the first partition member is lowered;
a step of raising the first partition member and dispersing the cells cultured in the second culture zone into the first culture zone; and
a step of culturing cells in the first culture zone and the second culture zone.

8. A cell subculture method which uses a cell culture container (1) according to claim 2, the method comprising:
a step of culturing cells in the third culture zone in a state that the second partition member is lowered;
a step of raising the second partition member in a state that the first partition member is lowered and dispersing the cells cultured in the third culture zone into the second culture zone;
a step of culturing cells in the third culture zone and the second culture zone in a state that the first partition member is lowered;
a step of raising the first partition member and dispersing the cells cultured in the third culture zone and the second culture zone into the first culture zone; and
a step of culturing cells in the first culture zone, the second culture zone, and the third culture zone.

9. A cell subculture method which uses the cell culture container (1) according to claim 3, the method comprising:
a step of culturing cells in the second culture zone and the third culture zone in a state that the first partition member and the third partition member are lowered;
a step of raising the first partition member and the third partition member and dispersing the cells cultured in the second culture zone and the third culture zone into the first culture zone; and
a step of co-culturing cells in the first culture zone, the second culture zone, and the third culture zone.

## Patentansprüche

1. Zellkulturbehälter, der Folgendes umfasst:
in einem versiegelbaren Kulturbehälter (1), der einen ersten Kulturbereich bildet,
ein erstes Teilungselement, das eine Wandfläche aufweist, die derart ausgebildet ist, dass sie niedriger als eine Wandfläche des Kulturbehälters (1) ist, das einen zweiten Kulturbereich bildet; und
Mittel zum Anheben und Absenken des ersten Teilungselements in einem Zustand, in dem der Kulturbehälter (1) abgedichtet ist.

2. Zellkulturbehälter nach Anspruch 1, der ferner Folgendes umfasst:
ein zweites Teilungselement, das eine Wandfläche aufweist, die derart ausgebildet ist, dass sie niedriger als eine Wandfläche des Kulturbehälters (1) ist, das im zweiten Kulturbereich einen dritten Kulturbereich bildet; und
Mittel zum Anheben und Absenken des zweiten Teilungselements in einem Zustand, in dem der Kulturbehälter (1) abgedichtet ist.

3. Zellkulturbehälter nach Anspruch 1, der ferner Folgendes umfasst:
ein drittes Teilungselement, das eine Wandfläche aufweist, die derart ausgebildet ist, dass sie niedriger als eine Wandfläche des Kulturbehälters (1) ist, das den dritten Kulturbereich bildet, der mit dem zweiten Kulturbereich nicht überlappt; und
Mittel zum Angeben und Absenken des dritten Teilungselements in einem Zustand, in dem der Kulturbehälter (1) abgedichtet ist.

4. Zellkulturbehälter nach einem der Ansprüche 1 bis 3,
wobei Magneten (4A, 6A, 14A, 15A, 16A) als die Mittel zum Anheben und Absenken des Teilungselements verwendet werden.

5. Zellkulturbehälter nach einem der Ansprüche 1 bis 3,
wobei ein mit dem Teilungselement verbundenes Trägerelement als das Mittel zum Anheben und Absenken des Teilungselements verwendet wird.

6. Automatische Subkulturvorrichtung, die Folgendes umfasst:
den Zellkulturbehälter (1) nach einem der Ansprüche 1 bis 5;
einen Zuführmechanismus für das Kulturmedium, der dem Kulturbehälter (1) ein Kulturmedium zuführt;
einen Zuführmechanismus für gemischtes Gas, der dem Kulturbehälter (1) ein gemischtes Gas zuführt; und
einen Zellbeobachtungsmechanismus zum Beobachten einer Kulturfläche des Kulturbehälters (1).

7. Zellsubkulturverfahren, das den Zellkulturbehälter (1) nach Anspruch 1 verwendet, wobei das Verfahren Folgendes umfasst:
einen Schritt des Kultivierens von Zellen im zweiten Kulturbereich in einem Zustand, in dem das erste Teilungselement abgesenkt ist;
einen Schritt des Anhebens des ersten Teilungselements und des Verteilens der in der zweiten Kulturzone kultivierten Zellen im ersten Kulturbereich; und
einen Schritt des Kultivierens der Zellen im ersten Kulturbereich und im zweiten Kulturbereich.

8. Zellsubkulturverfahren, das einen Zellkulturbehälter (1) nach Anspruch 2 verwendet, wobei das Verfahren Folgendes umfasst:
einen Schritt des Kultivierens von Zellen im dritten Kulturbereich in einem Zustand, in dem das zweite Teilungselement abgesenkt ist;
einen Schritt des Anhebens des zweiten Teilungselements in einem Zustand, in dem das erste Teilungselement abgesenkt ist, und des Verteilens der im dritten Kulturbereich kultivierten Zellen im zweiten Kulturbereich;
einen Schritt des Kultivierens der Zellen im dritten Kulturbereich und im zweiten Kulturbereich in einem Zustand, in dem das erste Teilungselement abgesenkt ist;
einen Schritt des Anhebens des ersten Teilungselements und des Verteilens der im dritten Kulturbereich und im zweiten Kulturbereich kultivierten Zellen im ersten Kulturbereich; und
einen Schritt des Kultivierens der Zellen im ersten Kulturbereich, im zweiten Kulturbereich und im dritten Kulturbereich.

9. Zellsubkulturverfahren, das den Zellkulturbehälter (1) nach Anspruch 3 verwendet, wobei das Verfahren Folgendes umfasst:
einen Schritt des Kultivierens von Zellen im zweiten Kulturbereich und im dritten Kulturbereich in einem Zustand, in dem das erste Teilungselement und das dritte Teilungselement abgesenkt sind;
einen Schritt des Anhebens des ersten Teilungselements und des dritten Teilungselements und des Verteilens der im zweiten Kulturbereich und im dritten Kulturbereich kultivierten Zellen im ersten Kulturbereich; und
einen Schritt des gemeinsamen Kultivierens der Zellen im ersten Kulturbereich, im zweiten Kulturbereich und im dritten Kulturbereich.

## Revendications

1. Récipient pour cultures cellulaires, comprenant :
dans un récipient pour cultures (1) capable d'être scellé, qui forme une première zone de culture
un premier élément de cloisonnement ayant une surface de paroi formée pour être plus basse qu'une surface de paroi du récipient pour cultures (1), qui forme une seconde zone de culture ; et
un moyen pour faire monter et descendre le premier élément de cloisonnement dans un état dans lequel le récipient pour cultures (1) est scellé.

2. Récipient pour cultures cellulaires selon la revendication 1, comprenant en outre :
un second élément de cloisonnement ayant une surface de paroi formée pour être plus basse qu'une surface de paroi du récipient pour cultures (1), qui forme une troisième zone de culture dans la seconde zone de culture ; et
un moyen pour faire monter et descendre le second élément de cloisonnement dans un état dans lequel le récipient pour cultures (1) est scellé.

3. Récipient pour cultures cellulaires selon la revendication 1, comprenant en outre :
un troisième élément de cloisonnement ayant une surface de paroi formée pour être plus basse qu'une surface de paroi du récipient pour cultures (1), qui forme la troisième zone de culture qui ne chevauche pas la seconde zone de culture ; et
un moyen pour faire monter et descendre le troisième élément de cloisonnement dans un état dans lequel le récipient pour cultures (1) est scellé.

4. Récipient pour cultures cellulaires selon l'une quelconque des revendications 1 à 3,
dans lequel des aimants (4A, 6A, 14A, 15A, 16A) sont utilisés à titre de moyen pour faire monter et descendre l'élément de cloisonnement.

5. Récipient pour cultures cellulaires selon l'une quelconque des revendications 1 à 3,
dans lequel un élément de support connecté à l'élément de cloisonnement est utilisé à titre de moyen pour faire monter et descendre l'élément de cloisonnement.

6. Dispositif de sous-culture automatique, comprenant :
le récipient pour cultures cellulaires (1) selon l'une quelconque des revendications 1 à 5 ;
un mécanisme d'alimentation de milieu de culture qui alimente un milieu de culture au récipient pour cultures (1) ;
un mécanisme d'alimentation de mélange gazeux qui alimente un mélange gazeux dans le récipient pour cultures (1) ; et
un mécanisme d'observation de cellules pour observer une surface de culture du récipient pour cultures (1).

7. Procédé de sous-culture de cellules qui utilise le récipient pour cultures cellulaires (1) selon la revendication 1, le procédé comprenant :
une étape consistant à cultiver des cellules dans la seconde zone de culture dans un état dans lequel le premier élément de cloisonnement est descendu ;
une étape consistant à faire monter le premier élément de cloisonnement et disperser les cellules cultivées dans la seconde zone de culture vers la première zone de culture ; et
une étape consistant à cultiver des cellules dans la première zone de culture et dans la seconde zone de culture.

8. Procédé de sous-culture de cellules qui utilise un récipient pour cultures cellulaires (1) selon la revendication 2, le procédé comprenant :
une étape consistant à cultiver des cellules dans la troisième zone de culture dans un état dans lequel le second élément de cloisonnement est descendu ;
une étape consistant à faire monter le second élément de cloisonnement dans un état dans lequel le premier élément de cloisonnement est descendu, et à disperser les cellules cultivées dans la troisième zone de culture vers la seconde zone de culture ;
une étape consistant à cultiver des cellules dans la troisième zone de culture et dans la seconde zone de culture dans un état dans lequel le premier élément de cloisonnement est descendu ;
une étape consistant à faire monter le premier élément de cloisonnement et à disperser les cellules cultivées dans la troisième zone de culture et dans la seconde zone de culture vers la première zone de culture ; et
une étape consistant à cultiver des cellules dans la première zone de culture, dans la seconde zone de culture, et dans la troisième zone de culture.

9. Procédé de sous-culture de cellules qui utilise le récipient pour cultures de cellules (1) selon la revendication 1, le procédé comprenant :
une étape consistant à cultiver des cellules dans la seconde zone de culture et dans la troisième zone de culture dans un état dans lequel le premier élément de cloisonnement et le troisième élément de cloisonnement sont descendus ;
une étape consistant à faire monter le premier élément de cloisonnement et le troisième élément de cloisonnement et à disperser les cellules cultivées dans la seconde zone de culture et dans la troisième zone de culture vers la première zone de culture ; et
une étape consistant à cultiver conjointement des cellules dans la première zone de culture, dans la seconde zone de culture, et dans la troisième zone de culture.
